(19) Europäisches Patentamt
European Patent Office
Office européen des brevets

(11) Publication number: **0 182 609**
**B1**

(12) # EUROPEAN PATENT SPECIFICATION

(45) Date of publication of patent specification: **09.05.90**

(51) Int. Cl.[5]: **C 07 C 215/26,**
C 07 C 47/546, A 61 K 31/13

(21) Application number: **85308280.8**

(22) Date of filing: **14.11.85**

(54) **Polycyclic biocidal compounds, their synthesis, formulations containing them.**

(30) Priority: **15.11.84 GB 8428932**

(43) Date of publication of application:
**28.05.86 Bulletin 86/22**

(45) Publication of the grant of the patent:
**09.05.90 Bulletin 90/19**

(84) Designated Contracting States:
**AT BE CH DE FR GB IT LI LU NL SE**

(56) References cited:
**EP-A-0 125 701**
**EP-A-0 125 702**

(73) Proprietor: **THE WELLCOME FOUNDATION LIMITED**
**183-193 Euston Road**
**London NW1 2BP (GB)**

(72) Inventor: **Bair, Kenneth Walter**
**342 Wesley Drive**
**Chapel Hill North Carolina 27514 (US)**

(74) Representative: **Rollins, Anthony John et al**
**Group Patents & Agreements The Wellcome Foundation Ltd Langley Court**
**Beckenham Kent BR3 3BS (GB)**

**EP 0 182 609 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to polycyclic alkanol derivatives which have been found to have biocidal activity. More specifically the invention concerns aminoalkanol derivatives containing a polycarbocyclic ring system, methods for the synthesis thereof, pharmaceutical formulations thereof, pharmaceutical formulations thereof and the use thereof as biocidal agents, particularly antitumor agents.

*Gazz. Chim. Ital. 93,* 118 (1963) reports the preparation of 2-phenylmethylamino-2-methyl-1,3-propanediol, but no antitumor activity is disclosed for this compound. Two analogues of nitracrine (1-nitro-9-((3-dimethylaminopropyl)amino)-acridine) containing 2-amino-2-methyl-1,3-propanediol and tris-(hydroxymethyl)methylamine groups have shown antitumor activity in murine screening systems *(Arzneim. Forsch./Drug Res. 32II,* 1013 (1982)).

EP—A—0 125 701 and EP—A—0 125 702 report perylene, fluoranthene, chrysene, pyrene and triphenylene amino-alkanol derivatives having biocidal and particularly antitumour activity.

We have now discovered a novel class of polycarbocyclic armoatic alkanol derivatives which have biocidal activity.

Accordingly, in a first aspect, the present invention provides a compound of the formula (I)

$$RCH_2NHR^1 \qquad (I)$$

or a monomethyl or monoethyl ether thereof, the compound of formula (I) including the said ethers containing no more than 30 carbon atoms in total; an ester thereof; a salt thereof;

wherein R is a $C_{15-22}$ carbocylic fused ring system containing 3, 4 or 5 aromatic rings optionally substituted by one or two substituents, said substituents containing not more than four carbon atoms in total when taken together being the same or different and are selected from halogen; cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(O)_nR^2$ wherein n is an integer O, 1 or 2 and $R^2$ is $C_{1-2}$ alkyl optionally substituted by hydroxy or $C_{1-2}$ alkoxy; or R is optionally substituted by a group $NR^3R^4$ containing not more than 5 carbon atoms where $R^3$ and $R^4$ are the same or different and each is a $C_{1-3}$ alkyl group or $NR^3R^4$ forms a five- or six-membered heterocyclic ring optionally containing one or two additional heteroatoms;

$R^1$ contains not more than eight carbon atoms and is a group

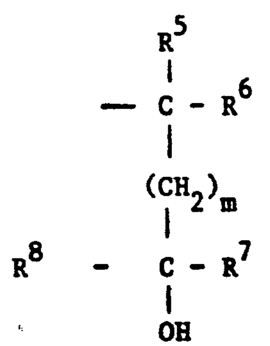

or

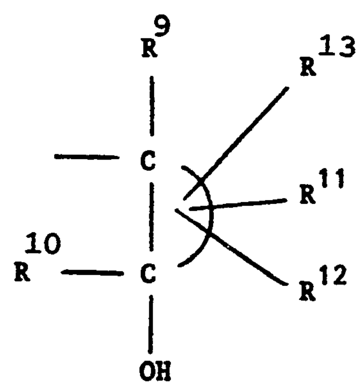

wherein

m is 0 or 1;

$R^5$ and $R^6$ are the same or different and each is hydrogen or $C_{1-5}$ alkyl optionally substituted by hydroxy;

$R^7$ and $R^8$ are the same or different and each is hydrogen or $C_{1-3}$ alkly; $-\overset{\frown}{C-C}-$ is a five- or six-membered saturated carbocyclic ring;

$R^9$ is hydrogen, methyl or hydroxymethyl;

$R^{10}$, $R^{11}$ and $R^{12}$ are the same or different and each is hydrogen or methyl;

$R^{13}$ is hydrogen, methyl, hydroxy, or hydroxymethyl;

with the proviso that R is not substituted or unsubstituted perylene, fluoranthene, chrysene, pyrene or triphenylene.

Preferably, the fused ring system R is planar or deviates only slightly from planarity. Thus, the fused ring system suitably contains a maximum of two non-aromatic carbon atoms which may be in the same ring, in which case they are adjacent, or in different carbocyclic rings. Preferably, there is a maximum of one non-aromatic carbon atom in the ring system or two non-aromatic carbon atoms in the same ring.

Suitably the fused carbocyclic ring system will contain between 16 and 20 carbon atoms and preferably 16 to 18 carbon atoms. Preferred ring systems include the following:

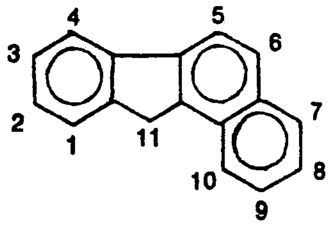

11*H*-benzo[*a*]fluorene
$C_{17}$

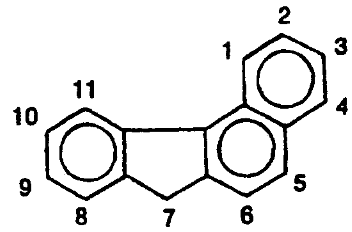

7*H*-benzo[*c*]fluorene
$C_{17}$

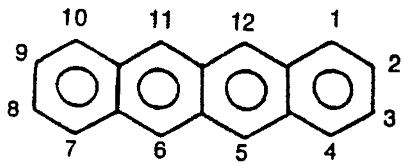

naphthacene $C_{18}$

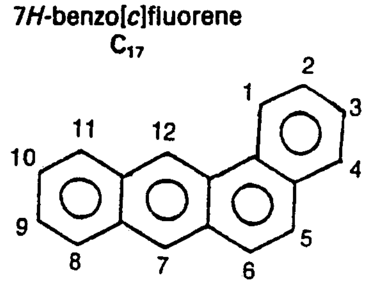

$C_{18}$
benz[*a*]anthracene

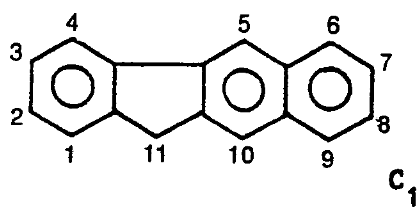

$C_{17}$
11H-benzo[*b*]fluorene

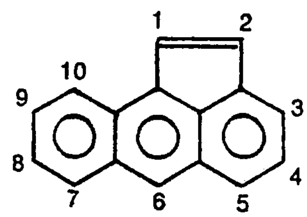

$C_{16}$
aceanthrylene

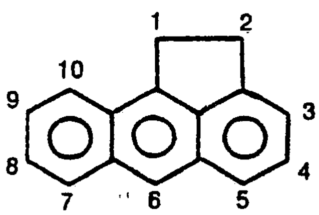

$C_{16}$
aceanthrene

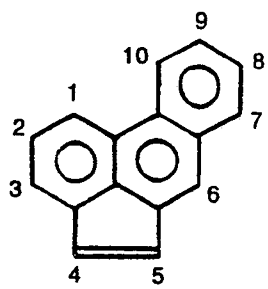

$C_{16}$
acephenanthrylene

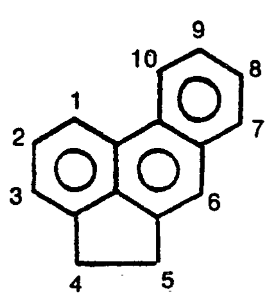

$C_{16}$

acephenanthrene

Preferred compounds include those wherein R is naphthacene, benz[*a*]anthracene, 11*H*-benzo[*b*]fluorene, acephenanthrylene or acephenanthrene. One preferred group of compounds of the formula (I) includes those compounds wherein the side chain $CH_2NHR^1$ is attached to the ring atom of R which has the highest electron density when R is unsubstituted. Thus, particularly preferred compounds include those wherein R is 7-benz[*a*]anthracene, 5-naphthacene, 5-(11*H*-benzo[*b*]fluorene), 6-acephenanthrylene or 6-acephenanthrene.

Suitably $RCH_2R^1$ or a monomethyl or monethyl ether thereof contains not more than 28 carbon atoms in total.

Suitably m i 0.

Suitably $R^1$ is

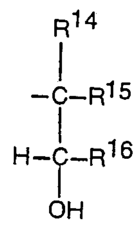

or

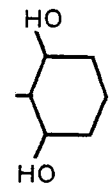

wherein

$R^{14}$ is $CH_2OH$, $CH(CH_3)OH$ or $CH_2CH_2OH$;

$R^{15}$ is hydrogen, $C_{1-3}$ alkyl or $CH_2OH$;

$R^{16}$ is hydrogen or methyl.

Preferably $R^{14}$ is $CH_2OH$ or $CH(CH_3)OH$. Preferably $R^{15}$ is hydrogen, methyl, ethyl or $CH_2OH$.

Most preferably $R^1$ is

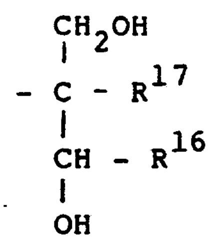

wherein $R^{16}$ is hydrogen or methyl and $R^{17}$ is hydrogen, methyl or ethyl, most preferably methyl.

Preferred compounds of the formula (I) include:

2-[(7-Benz[*a*]anthracenylmethyl)aminol]-2-methyl-1,3-propanediol,

2-[(5-Naphthacenylmethyl)amino]-2-methyl-1,3-propanediol,

2-[(11*H*-Benzo[*b*]fluoren-5-ylmethyl)amino]-2-methyl-1,3-propanediol,

2-[(6-Acephenanthrylenylmethyl)amino]-2-methyl-1,3-propanediol and

2-[(6-Acephenanthrenylmethyl)amino]-2-methyl-1,3-propanediol ethers, esters thereof; acid addition salts thereof.

Salts included within the scope of the present invention are those of compounds of formula (I) and ethers and esters thereof.

Esters and non-pharmaceutically useful salts of the compounds of the formula (I) are useful intermediates in the preparation and purification of compounds of the formula (I) and pharmaceutically useful salts thereof, and are therefore within the scope of the present invention. Thus, salts of the compounds of the formula (I) useful in the present invention include but are not limited to those derived from inorganic acids, such as hydrochloric, hydrobromic, sulfuric and phosphoric acids, and organic acids such as isethionic (2-hydroxyethylsulfonic), maleic, malonic, succinic, salicylic, tartaric, lactic, citric, formic, lactobionic, pantothenic, methanesulfonic, ethanesulfonic, benzenesulfonic, *p*-toluenesulfonic, naphthalene-2-sulfonic, and ascorbic acids, and amino acids such as glycine.

Pharmacologically and pharmaceutically acceptable salts are preferred, particularly those that are soluble in solvents suitable for parenteral administration, for example, hydrochlorides, methanesulfonates and isethionates.

Esters of compounds of formula (I) are derived from acids known to those skilled in the art to be suitable for ester formation, and are conveniently those derived from $C_{1-6}$ alkanoic acids or alkanoic acid derivatives, for example acetic acid, propionic acid, *n*-butyric acid and *iso*-butyric acid. The esters may be formed from all or only some of the hydroxy groups contained in the compounds a formula (I).

The compounds of formula (I) and their ethers, esters, and salts thereof may be prepared by any method known in the art for the preparation of compounds of analogous structure. Thus, the compounds of formula (I) may, for example, be prepared by any of the methods defined below.

1. The reduction of a compound $RCH{=}NR^1$ wherein R and $R^1$ are as hereinbefore defined, or an appropriately protected derivative thereof followed by deprotection where appropriate.

The conditions and reagents for such a reaction are well known to those skilled in the art, and any such conditions/reagents may be employed. The conversion of (II) or suitably protected derivatives thereof may be carried out by a reducing agent followed by deprotection if necessary. The reduction is conveniently carried out by a metal hydride such as lithium aluminum hydride, sodium borohydride, sodium cyanoborohydride, or by catalytic hydrogenation, conveniently by hydrogen in the presence of a metal catalyst such as palladium or platinum, or equivalent reagents as outlined by J. March, *Advanced Organic Chemistry*, 2nd ed., pages 819—820, McGraw Hill, New York, 1977. The reduction is suitably carried out with the compound of formula (II) in solution in an inert solvent or mixture of solvents compatible with the reducing agent, at a non-extreme temperature, for example, between 0° and 80°C, conveniently at room temperature.

In the case of lithium aluminum hydride and like reagents, suitable solvents include ethers (for example tetrahydrofuran, diethyl ether and 1,2-dimethoxyethane) optionally in the presence of a hydrocarbon cosolvent (for example toluene, benzene or hexane).

In the case of sodium borohydride and like reagents, suitable solvents include alcohols (for example ethanol, methanol or isopropanol), optionally in the presence of a hydrocarbon cosolvent (for example toluene, benzene or hexane) or an ether cosolvent (for example diethylether or tetrahydrofuran).

In the case of sodium cyanoborohydride and like reagents, suitable solvents include those described or sodium borohydride and the reduction is conveniently carried out in the presence of an acid, conveniently glacial acetic acid or ethanolic hydrochloric acid as outlined in, for example, R. Hutchins *et al., Organic Preparations and Procedures International 11*, 201 (1979).

In the case of catalytic hydrogenation, suitable solvents include alcohols (for example methanol and ethanol) optionally in the presence of a hydrocarbon cosolvent (for example toluene or benzene), or ether cosolvent (for example diethyl ether or tetrahydrofuran) optionally in the presence of an acid (for example glacial acetic acid or ethanolic hydrochloric acid), or glacial acetic acid.

Protected derivatives of compounds $RCH{=}NR^1$ are conveniently used when lithium aluminum hydride is employed as the reducing agent. Convenient protecting groups are compatible with the reducing agent utilized and are readily removed under nondestructive conditions, for example benzyl, tetrahydropyranyl and isopropylidene ethers.

It is often convenient not to isolate the compound $RCH{=}NR^1$ but to react a compound RCHO with a compound $NH_2R^1$; wherein R and $R^1$ are as defined in (I) and to reduce the compound of the formula $RCH{=}NR^1$ so formed *in situ*. The reaction of the compounds RCHO and $NH_2R^1$ is again suitably carried out using conditions and reagents which are well known to those skilled in the art, for example in the presence of an acid, such as a sulfonic acid, i.e. *p*-toluenesulfonic acid, in an appropriate inert solvent, such as an aromatic hydrocarbon, suitably toluene, with azeotropic removal of water followed by treatment with the reducing agent in an appropriate solvent, suitably ethanol or methanol.

Alternatively $RCH{=}NR^1$ formed under equilibrium conditions in appropriate solvents can be reduced *in situ* with an appropriate reducing agent, suitably sodium cyanoborohydride.

The compound RCHO may be in the form of a protected aldehyde, for example an acetal, which liberates the aldehyde function under the reaction conditions. In turn, a compound RCHO can be synthesized by reacting the appropriate polycyclic aromatic hydrocarbon with a formylating agent such as that generated by the reaction between $ScCl_4$ and $Cl_2CHOCH_3$ or equivalent reagents, for example, according to the method of A. Reiche *et al., Chem. Ber. 93,* 88 (1960), or with other standard formylating reagents/procedures known to the art, for example, the Gatterman-Koch reaction ($CO/HCl/AlCl_3/CuCl$), the Gatterman reaction ($HCN/HCl/ZnCl_2$), and the Vilsmeier reaction ($POCl_3/PhN(Me)CHO$ or $POCl_2/Me_2NCHO$) (J. March, *vide supra* pages 494—497).

The compounds RCHO may also be prepared from an appropriate polycyclic aromatic hydrocarbon substituted by a suitable functional group such as $CH_2OH$, $CHBr_2$, $CH_3$, $COCH_3$, COOH, or CN, and converting this functional group to an aldehyde group by methods well known to those skilled in the art.

Where the polycyclic aromatic ring bears *substituents,* RCHO may be prepared by a variety of methods known in the art of organic chemistry depending on the nature of the substituent on the ring. For example, if the substituent(s) is a halogen, the starting materials may be prepared by direct treatment of the polycyclic aromatic hydrocarbon with a halogenating agent (e.g. $Cl_2$, $Br_2$, or $SO_2Cl_2$) or indirectly by such routes as the Sandmeyer reaction (H. H. Hodgson, *Chem. Rev. 40,* 251 (1947)). If the substituent(s) is alkyl, the polycyclic aromatic hydrocarbon may be reacted with the appropriate reagents under Friedel-Crafts reaction conditions (G. A. Olah, *Friedel Crafts and Related Reactions*, Vols. 1—3, Interscience, New York, NY, 1963—1965).

The compounds of the formula $NH_2R^1$ and ethers thereof also may be prepared by methods known in the art, for example, when $R^1$ is as hereinbefore defined, by the reaction of $NO_2R^{18}$, wherein $R^{18}$ is

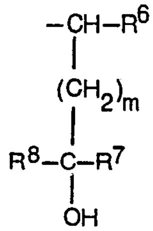

wherein $R^6$ to $R^8$ and m are as hereinbefore defined, with an appropriate aldehyde, conveniently acetaldehyde or formaldehyde (as in B. M. Vanderbilt and H. B. Hass, *Ind. Eng. Chem. 32,* 34 (1940)) followed by reduction (as outlined in J. March, *vide supra,* pages 1125—1126), conveniently by hydrogen and a metal catalyst (for example, a platinum containing catalyst) in an appropriate solvent, conveniently glacial acetic acid.

2. The reduction of a compound $R.CO.NHR^1$ wherein R and $R^1$ are as hereinbefore defined and the hydroxy groups are optionally protected, followed by deprotection of the hydroxy groups where appropriate. The reduction may be carried out by standard reducing agents known for carrying out this type of reduction (as outlined in J. March, *vide supra*, page 1122), for example, a hydride reagent such as lithium aluminium hydride in an inert solvent, such as an ether, i.e. tetrahydrofuran, at a non-extreme temperature, for example, at between 0° and 100°C and conveniently at the reflux temperature of the ether.

The compound $R.CO.NHR^1$ may be formed by the reaction of the appropriate acid (RCOOH) or a suitable reactive acid derivative thereof as outlined in J. March, *vide supra*, pages 382—390) for example, an acid halide in an inert solvent, with an amine $NH_2R^1$ in which the hydroxy groups are optionally protected, for example, when the compound $NH_2R^1$ is a diol, by an isopropylidene group. The compound $R.CO.NHR^1$ so formed is suitably reduced *in situ* and deprotected if necesary to give a compound of formula (I). The compounds of the formula RCOOH can be prepared by methods well known to those skilled in the art.

3. The reaction of a compound $RCH_2L$ (wherein Ar is as hereinbefore defined and L is a leaving group), with a compound $NH_2R^1$ as hereinbefore defined. Suitable leaving groups are those defined by J. March, *vide supra* pages 325—331, and include halogens such as chlorine or bromine and sulfonic acid derivatives such as *p*-toluenesulfonate. The reaction is suitably carried out in an appropriate solvent, such as a dipolar aprotic solvent or alcohol at a non-extreme temperature, for example 50—100°. The compounds of the formula $RCH_2L$ can be prepared by methods well known to those skilled in the art.

There is therefore provided, as a further aspect of the invention, a method for the preparation of a compound of formula (I) comprising in particular those methods defined in (1) to (3) hereinabove.

The compounds of this invention have biocidal activity, e.g. are toxic to certain living cells which are detrimental to animals such as mammals and poultry, for example pathogenic organisms and tumours. Compounds of this invention are also toxic to organisms which are pathogenic in or detrimental to plants and plant materials.

This toxicity to pathogenic organisms have been demonstrated by activity against one or more of the following: viruses (e.g. *Herpes simplex* I/vero), fungi (e.g. *Microsporum* sp., *Fusarium solani* and *trychophyton* sp.) protozoa (e.g. *Eimeria tenella* and *Trichomonas vaginalis*, bacteria (e.g. *Mycoplasma smegmatis and Streptococcus pyogenes*), and helminths (e.g. *Nippostrongylus brasiliensis* and *Brugia pahangi*). The antitumour activity of compounds of formula I have been demonstrated in a number of recognized screens and primarly by activity against ascitic P388/O leukaemia.

Preferred compounds of the formula (I) are those which have antitumour activity. The activity against ascitic tumours, including P388/O, is evidenced by reduction of tumour cell number in mammals (for example, mice bearing ascitic tumours) and their consequent increase in survival duration as compared to an untreated tumour bearing control group. Antitumour activity is further evidenced by measurable reduction in the size of solid tumours following treatment of mammals with the compounds of this invention compared to the tumours of untreated control tumour bearing animals. Compounds of formula (I) are active against murine tumours such as lymphocytic leukaemia P388/O, lymphocytic leukaemia L1210, melanotic melanoma B16, P815 mastocytoma, MDAY/D2 fibrosarcoma, colon 38 adenocarcinoma, M5076 rhabdomyosarcoma and Lewis lung carcinoma.

Activity in one or more of these tumour tests has been reported to be indicative of antitumour activity in man (A. Goldin *et al.* in *Methods in Cancer Research* ed. V. T. DeVita Jr. and H. Busch, *16*, 165, Academic Press, N.Y. 1979).

There are sublines of P388/O which have been made resistant to the following clinically useful agents: cytosine arabinoside, doxorubicin, cyclophosphamide, *L*-phenylalanine mustard, methotrexate, 5-fluorouracil, actinomycin D, *cis*-platin and bis-chloroethylnitrosourea. Compounds of this invention show potent activity against these drug-resistant tumours using the procedure for P388/O above.

Compounds of formula (I) have also been found to be active against human tumour cells in primary cultures of lung, ovary, breast, renal, melanoma, unknown primary, gastric, pancreatic, mesothelioma, myeloma, and/or colon cancer. (As used herein "cancer" is to be taken as synonymous with "malignant tumour" or more generally "tumour" unless otherwise noted.) This is a procedure in which the prevention

of tumour cell colony formation, i.e. tumour cell replication, by a drug has been shown to correlate with clinical antitumour activity in man (D. D. Von Hoff *et al., Cancer Chemotherapy* and *Pharmacology* 6, 265 (1980); S. Salmon and D. D. Von Hoff, *Seminars* in *Oncology,* 8, 377 (1981)).

Compounds of formula I which have been found to have antitumour activity intercalate *in vitro* with DNA (this property is determined by viscometric methods using the procedure of W. D. Wilson *et al., Nucleic Acids Research* 4, 2697 (1954)) and have a log P as calculated by the method of C. Hansch and A. Leo in *Substituent Constants for Correlation Analysis in Chemistry and Biology,* John Wiley and Sons, New York, 1979, lying in the range between −2.0 and +2.5.

As has been described above the compounds of the present invention are useful for the treatment of tumours. The invention thus further provides a method for the treatment of tumours in animals, including mammals, especially humans, which comprises the administration of a clinically useful amount of compound of formula (I) in a pharmaceutically useful form, once or several times a day or other appropriate schedule, orally, rectally, parenterally, or applied topically.

In addition, there is provided as a further, or alternative, aspect of the invention, a compound of formula (I) for use in therapy, for example as an antitumour agent.

In a still further aspect, there is provided a compound of the formula (I) for use against pathogenic organisms, for example fungi.

The amount of compound of formula (I) required to be effective as a biocidal agent will, of course, vary and is ultimately at the discretion of the medical or veterinary practitioner. The factors to be considered include the condition being treated, the route of administration, and nature of the formulation, the mammal's body weight, surface area, age and general condition, and the particular compound to be administered. A suitable effective antitumour dose is in the range of about 0.1 to about 120 mg/kg body weight, preferably in the range of about 1.5 to 50 mg/kg, for example 10 to 30 mg/kg. The total daily dose may be given as a single dose, multiple doses, *e.g.*, two to six times per day or by intravenous infusion for selected duration. For example, for a 75 kg mammal, the dose range would be about 8 to 9000 mg per day, and a typical dose would be about 2000 mg per day. If discrete multiple doses are indicated, treatment might typically be 500 mg of a compound of formula I given 4 times per day in a pharmaceutically useful formulation.

Whilst it is possible for the active compound (defined herein as compound of formula (I), or ether, ester, or salt thereof) to be administered alone, it is preferable to present the active compound in a pharmaceutical formulation. Formulations of the present invention, for medical use, comprise an active compound together with one or more pharmaceutically acceptable carriers thereof and optionally other therapeutical ingredients. The carrier(s) must be pharmaceutically acceptable in the sense of being compatible with the other ingredients of the formulation and not deleterious to the recipient thereof.

The present invention, therefore, further provides a pharmaceutical formulation comprising a compound of formula (I) (in the form of the free base, ether, or ester derivative or a pharmaceutically acceptable acid addition salt thereof) together with a pharmaceutically acceptable carrier therefor.

There is also provided a method for the preparation of a pharmaceutical formulation comprising bringing into association a compound of formula (I) an ether, ester, or pharmaceutically acceptable salt thereof, and a pharmaceutically acceptable carrier therefor.

Whilst the antitumour activity of the compounds of formula (I) is believed to reside in the free base, it is often convenient to administer an acid addition salt of a compound of formula (I).

The formulations include those suitable for oral, rectal or parenteral (including subcutaneous, intramuscular and intravenous) administration. Preferred formulations are those suitable for oral or parenteral administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a liquid carrier or a finely divided solid carrier or both and then, if necessary, shaping the product into desired formulations.

Formulations of the present invention suitable for oral administration may be presented as discrete units such as capsules, cachets, tablets or lozenges, each containing a predetermined amount of the active compound; as a powder or granules; or a suspension in an aqueous liquid or non-aqueous liquid such as a syrup, an elixir, an emulsion or a draught.

A tablet may be made by compression or moulding, optionally with one or more accessory ingredients. Compressed tablets may be prepared by compressing in a suitable machine the active compound in a free-flowing form such as a powder or granules, optionally mixed with a binder, lubricant, inert diluent, surface active or dispersing agent. Moulded tablets may be made by moulding in a suitable machine, a mixture of the powdered active compound with any suitable carrier.

A syrup may be made by adding the active compound to a concentrated, aqueous solution of a sugar, for example sucrose, to which may also be added any accessory ingredients. Such accessory ingredient(s) may include flavourings, an agent to retard crystallization of the sugar or an agent to increase the solubility of any other ingredient, such as a polyhydric alcohol for example glycerol or sorbitol.

Formulations for rectal administration may be presented as a suppository with a conventional carrier

such as cocoa butter.

Formulations suitable for parenteral administration conveniently comprise a sterile aqueous preparation of the active compound which is preferably isotonic with the blood of the recipient. Such formulations suitably comprise a solution of a pharmaceutically and pharmacologically acceptable acid addition salt of a compound of the formula (I) that is isotonic with the blood of the recipient. Thus, such formulations may conveniently contain distilled water, 5% dextrose in distilled water or saline and a pharmaceutically and pharmacologically acceptable acid addition salt of a compound of the formula (I) that has an appropriate solubility in these solvents, for example by hydrochloride, isethionate and methanesulfonate salts, preferably the latter.

Useful formulations also comprise concentrated solutions or solids containing the compound of formula (I) which upon dilution with an appropriate solvent give a solution suitable for parenteral administration as above.

In addition to the aforementioned ingredients, the formulations of this invention may further include one or more accessory ingredient(s) selected from diluents, buffers, flavouring agents, binders, surface active agents, thickeners, lubricants, preservatives (including antioxidants) and the like.

The following examples are provided by the way of illustration of the present invention and should in no way be construed as a limitation thereof.

General Comments

All solvents were reagent grade and used without further purification with the following exceptions. Tetrahydrofuran (THF) was dried by distillation from Na/K alloy under nitrogen ($N_2$) and used immediately. Toluene ($PhCH_3$) was distilled from $CaH_2$ under $N_2$ and stored over 3A molecular sieves. Chemicals used were reagent grade and used without purification unless noted. The full name and address of the suppliers of the reagents and chemicals is given when first cited. After this, an abbreviated name is used.

Preparative HPLC was carried out on a Waters Prep LC/System 500A machine using two 500 g silica gel ($SiO_2$) cartridges unless otherwise noted. Plus of $SiO_2$ used for purifications were "flash chromatography" silica gel (Merck Co., Inc., Merck Chemical Division, Rahway, NJ, 07065 silica gel 60, 230—400 mesh). An appropriate volume sintered glass funnel was filled approximately $\frac{3}{4}$ full with the silica gel and packed evenly by tapping the outside of the funnel. A piece of filter paper was then placed on top of the silica gel and a solution of the material to be purified applied evenly to the top. Gentle suction through a filter flask moved the eluting solvent through the plug rapidly. The appropriate size fractions were combined as needed and further manipulated.

General procedures are described in detail. Analogous procedures show melting point (mp), recrystallization solvents, and elemental analyses (all elements analyzing within a difference of ±0.4% of the expected value). Any changes to the procedure such as solvent, reaction temperature, reaction time, or workup are noted.

NMR ($^1H$, $^{13}C$), IR, MS data of all new products were consistent with the expected and proposed structures. The positions assigned to structural isomers were unequivocally determined by a number of NMR techniques. All final products were dried in a vacuum oven at 20 mm Hg pressure at the temperature indicated overnight (12—16 hours). All temperatures are in degrees Celsius.

Example 1

2-((7-Benz[*a*]anthracenylmethyl)amino)-2-methyl-1,3-propanediol methanesulfate

To a round-bottomed flask equipped with magnetic stiring bar, condenser, thermometer, Dean-Stark trap, and nitrogen inlet with bubbler was added benz[*a*]anthracene-7-carbaldehyde (Cambridge Chemical, Inc., 202 E. Smith St., Milwaukee, WI, 53207, 5.13 g, 20 mmol), 2-amino-2-methyl-1,3-propanediol (Aldrich Chemical Co., Milwaukee, WI, 53201, 4.20 g, 40 mmol), *p*-toluenesulfonic acid monohydrate (Aldrich, 0.1 g), and toluene (250 mL). The mixture was stirred at reflux with removal of water for 3 hours (or in the case of other examples until no further water is collected). Most of the toluene was then removed by distillation. The mixture was then cooled in an ice bath and diluted with abs. ethanol (200 mL) and further cooled. Solid sodium borohydride (MCB Manufacturing Chemists, Inc., 2909 Highland Ave., Cincinnati, OH, 45212, 1.51 g, 40 mmol) was added in one portion to the reaction mixture. The ice bath was then removed, the reaction mixture allowed to warm to room temperature and further stirred overnight. The reaction was then basified with 1N sodium hydroxide (2 L). The white solid which formed was filtered, washed with water (4 L), and sucked semidry. The solid was then dissolved in a mixture of abs. ethanol and methanesulphonic acid (99.5%, Morton Thiokol, Inc. — Alfa Products, PO Box 299, 152 Andover Street, Danvers, MA, 01923, 3 mL), filtered and diluted to 2 L with diethyl ether. After filtration, two additional crystallizations ($EtOH/Et_2O$) and drying, 7.48 g (84.7%) of 2-((7-benz[*a*]anthracenylmethyl)amino)-2-methyl-1,3-propanediol methanesulfonate was obtained mp 233—234° (dec), which analysed correctly (C, H, N, S) for the assigned structure.

Example 2

2[(Benzo[*a*]pyren-6-ylmethyl]amino]-2-methyl-1,3-propanediol methanesulfonate

Using the reductive amination procedure outlined in Example, 1 benzo[*a*]pyrene-6-carbaldehyde (Cambridge Chemical Inc.) and 2-amino-2-methyl-1,3,-propanediol (Aldrich) gave 2[(benzo[*a*]pyren-6-ylmethyl]amino]-2-methyl-1,3-propanediol methanesulfonate. 0.25 $H_2O$ in 53.1% yield mp darkens >195°,

275° (dec), which analysed correctly (C, H, N, S) for the assigned structure.

Example 3

2-[(5-Naphthacenylmethyl)amino]-2-methyl-1,3-propanediol

3A. 5-Naphthacenecarbaldehyde and

3B. 5,11-Naphthacenedicarbaldehyde

To a 3-neck round bottom flask equipped with overhead mechanical stirrer, condenser, addition funnel, nitrogen inlet tube and bubbler was added *N*-methyl-formanilide (Aldrich, 89.21 g, 0.66 mol, 81 mL) and O-dichlorobenzene (Aldrich, 200 mL). The mixture was cooled to 10° with an ice bath. Phosphorous oxychoride (Aldrich, 101.21 g, 0.66 mol, 60 mL) was then added dropwise to the solution not allowing the reaction temperature to rise above 10°. The reaction mixture was then allowed to warm to room temperature. Solid naphthacene (Cambridge Chemical, Inc., 50 g, 0.22 mol) was then added in five portions over 5 mins. The reaction mixture was warmed slowly to 80° and the mixture turned from purple to an almost black colour. The reaction was stirred at 80° for 12 hours, cooled, poured into ice water (2 L) and stirred an additional 12 hours at room temperature. The mixture was then filtered. The organic layer in the filtrate was applied to a plug of silica gel (500 g) and eluted first with toluene followed by methylene dichloride. The appropriate fractions were combined and the solvent removed by rotary evaporation. Recrystallization ($CH_2Cl_2$/EtOAc) gave 17.5 g of 5-naphthacenecarbaldehyde mp 163—164° (Lit. 164°, N. P. Buu-Hoi *et al, Recueil. 76,* 674, (1957)), which analysed correctly for the assigned structure (C. H). The solid was washed with a large volume of methanol (5 × 200 mL) dissolved in tetrahydrofuran (4 L) and passed through a plug of silica gel (500 g) using an additional 2 L of tetrahydrofuran as eluting solvent. The solvent was concentrated to a volume of 300 mL and then diluted to 800 mL with ethyl acetate. The solvent was concentrated to a volume of 250 mL and after filitration and drying 10.90 g of 5,11-naphthacenedicarbaldehyde mp 247—250°, was obtained which analysed correctly for the assigned structure (C, H).

3C. 2-[(5-Naphthacenylmethyl)amino]-2-methyl-1,3-propanediol methanesulfonate

Using the reductive amination procedure described in example, 1 5-naphthacenecarbaldehyde (3A) and 2-amino-2-methyl-1,3-propanediol (Aldrich) gave 2-[(5-naphthacenylmethyl)amino]-2-methyl-1,3-propanediol methanesulfonate mp 197.5—199°, (EtOH/$Et_2O$), which analysed correctly for the assigned structure (C, H, N, S).

Example 4

2-[(11*H*-Benzo[*b*]fluoren-5-ylmethyl)amino]-2-methyl-1,3-propanediol

4A. 11*H*-Benzo[*b*]fluorene-5-carbaldehyde.

11*H*-Benzo[*b*]fluorene (Cambridge Chemical, Inc.) was formylated using the procedure of A. Rieche *et al, Chem. Ber. 93,* 88 (1960). The crude aldehyde appeared to be mainly one isomer by TLC. The crude material was passed through a plug of silica gel, using toluene as the eluting solvent. Removal of the solvent and crystallization ($CH_2Cl_2$/hexane) gave 11*H*-benzo[*b*]fluorene-5-carbaldehyde in 86% yield, mp 104.5—106.5°, which analysed correctly for the assigned structure (C, H).

4B. 2[(11*H*-Benzo[*b*]fluoren-5-ylmethyl)amino]-2-methyl-1,3-propanediol methanesulfonate

Using the reductive amination precedure outlined in example 1, 11*H*-benzo[*b*]fluorene-5-carbaldehyde (4A) and 2-amino-2-methyl-1,3-propanediol (Aldrich) gave 2-[(11*H*-benzo[*b*]fluoren-5-ylmethyl)amino]-2-methyl-1,3-propanediol methanesulfonate in 49.6% yield, mp 211—213°, which analysed correctly for the assigned structure (C, H, N, S).

Example 5

2[(Benzo[*c*]phenanthren-5-ylmethyl]amino]-2-methyl-1,3-propanediol

5A. Benzo[*c*]phenanthrene-5-carbaldehyde.

Benzo[*c*]phenanthrene (Cambridge Chemical Inc.) was formylated using the procedure of A. Rieche *et al, Chem. Ber. 93,* 88 (1960). The crude aldehyde appeared to be mainly are isomer by TLC. The crude material was purified by chromatography ($SiO_2$, $PhCH_3$) followed by recrystallization ($CH_2Cl_2$/hexane). The pure material isolated in 74% yield was shown to be isomerically pure by NMR and was benzo[*c*]phenanthrene-5-carbaldehyde mp 133—134°. It analysed correctly for the assigned structure (C, H).

5B. 2[(Benzo[*c*]phenanthren-5-ylmethyl)amino]-2-methyl-1,3-propanediol methanesulfonate.

Using the reductive amination procedure described in example 1, benzo[*c*]phenanthrene-5-carbaldehyde (5A) and 2-amino-2-methyl-1,3-propanediol (Aldrich) gave 2-[(benzo[*c*]phenanthren-5-ylmethyl)amino]-2-methyl-1,3-propanediol methanesulfonate mp 205—207° (dec), (EtOH/$Et_2O$), which analysed correctly for the assigned structure (C, H, N, S).

Example 6

2-[(12-Chloro-5-naphthacenylmethyl)amino]-2-methyl-1,3-propanediol

6A. 12-Chloro-5-naphthacenecarbaldehyde

To a 3-neck round bottomed flask equipped with overhead stirrer, condenser, addition funnel, and

nitrogen inlet tube and bubbler was added 2,3-benz-9-anthrone (prepared by the procedure of L. F. Fieser *et al, J. Amer. Chem. Soc. 53,* 2329 (1931), 57.3 g, 0.235 mol) and dimethylformamide (Aldrich, 400 mL). The mixture was cooled to 0°. Phosphorus oxychloride (MCB, 167.5 g, 1.09 mol, 100 mL) was then added dropwise to the flask while the reaction temperature was maintained between 0—10° over a period of 1.5 hours. The reaction colour deepened to a dark purple as the reaction temperature was slowly raised to 75°. The reaction was stirred at this temperature for 2 hours, cooled and then stirred at room temperature overnight. The reaction mixture was poured into 2 L of a 2.8 M sodium acetate solution vigorously stirred for 30 min, then filtered. The dark purple solid was washed with water (1 L) then with methanol (3 × 500 mL). After drying in a vacuum oven (40°) overnight 63.9 g (97.6%) of 12-chloro-5-naphthacenecarbaldehyde was obtained which was used without further purification. An analytical sample was recrystallized ($CH_2Cl_2$/$CH_3OH$) mp 211.5—213.5°, and analysed correctly for the assigned structure (C, H).

6B. 2-[(12-Chloro-5-naphthacenylmethyl)amino]-2-methyl-1,3-propanediol methanesulfonate

Using the reductive amination procedure outlined in example 1, 12-chloro-5-naphthacenecarbaldehyde (6A) was reacted with 2-amino-2-methyl-1,3-propanediol (Aldrich) to give 2[(12-chloro-5-naphthacenylmethyl)amino]-2-methyl-1,3-propanediol methanesulfonate, mp 219—220° (dec), ($CH_3OH$/$Et_2O$), which analysed correctly for the assigned structure (C, H, N, Cl, S).

6C. 2-[[(6-Chloro-11-naphthacenylmethyl)amino]-2-methyl-1,3-propanediol hydrochloride

Using the reductive amination procedure outlined in example 1 except that the crude free base was dissolved in ethanolic hydrogen chloride, 12-chloro-5-naphthacenecarbaldehyde (6A) and 2-amino-2-methyl-1,3-propanediol (Aldrich) gave 2-[(12-chloro-5-naphthacenylmethyl)amino]-2-methyl-1,3-propanediol hydrochloride mp >300°, ($CH_3OH$/$Et_2O$), which analysed correctly for the assigned structure (C, H, N, Cl).

Antitumor Screening Results

Methods for evaluating the antitumour activity of these compounds are essentially those used in the Tumor Panel by the Developmental Therapeutics Program, Division of Cancer Treatment, National Cancer Institute, A. Goldin, *et al., Methods in Cancer Research,* Vol. XVI, p. 165, Academic Press (1979). Some modifications, in dose level and schedule have been made to increase the testing efficiency.

Lymphocytic Leukemia P338/O Test

CD2-$F_1$ mice, of the same sex, weighing 20 ± 3 g are used for this test. Control and test animals are injected intraperitoneally with a suspension of $-10^6$ viable P388/O tumor cells on day 0. In each test several dose levels which bracket the $LD_{20}$ for the compound are evaluated; each dose level group contains 6 animals. The test compounds are prepared either in physiologic saline containing 0.05% Tween 80 or distilled water containing 5% dextrose and are administered intraperitoneally on days 1, 5, and 9 relative to tumor implant. Doses are on a mg/kg basis according to individual animals' body weights. The day of death for each animal is recorded, the median identified for each group and the ratios of median survival time for treated (T)/control (C) groups are calculated. The criterion for activity is T/C × 100 >120%. Results of P388/O testing are summmarized in Table 1 below.

**Table I**

| Compound of Example | Optimal Dose (mg/kg) | T/C x 100% (Excluding 30 Day survivors | $LD_{20}$ (mg/kg) |
|---|---|---|---|
| 1 | 220 | +270 | 180 |
| 2 | 111 | +204 | 100 |
| 3C | 60 | +195 | 50 |
| 4B | 225 | +280 | 300 |
| 5B | 15 | +140 | 10 |
| 6B | 75 | +150 | 70 |
| 6C | 40 | +150 | 70 |

Example 7

2-((3-Benz[*a*]anthracenylmethyl)amino)-2-methyl-1,3-propanediol

7A. 5,6,8,9,10,11-Hexahydrobenz[*a*]anthracene-3-carbaldehyde

5,6,8,9,10,11-Hexahydrobenz[*a*]anthracene (Cambridge Chemicals, Inc. 38.0 g, 0.162 mol) was formlyated using the procedure of A. Rieche *et al* (*Chem. Ber. 93,* 88 (1960)). The crude aldehyde mixture was passed through a 40 × 10 cm plug of silica gel using toluene as the eluting solvent. Three aldehyde fractions were isolated. The least mobile of the fractions by TLC contained 11.23 g (26.5% yield) of crude 5,6,8,9,10,11-hexahydrobenz[*a*]anthracene-3-carbaldehyde (identified by $^1$H—NMR) which was used directly without purification.

7B. Benz[*a*]anthracene-3-carbaldehyde

To a round bottom flask equipped with magnetic stirring bar, reflux condenser and $N_2$ inlet line was added 5,6,8,9,10,11-hexahydrobenz[*a*]anthracene-3-carbaldehyde (7A, 10.41 g, 39.7 mmol), 2,3 dichloro-5,6-dicyano-1,4-benzoquinone (DDQ) (Aldrich, 27.02 g, 0.119 mol) and dry $PhCH_3$ (500 mL). The mixture was refluxed for 2 h until no more starting material remained by TLC, cooled and filtered to give a deep red solution. The solution was applied to a 40 × 10 cm column of silica and eluted with additional $PhCH_3$ as the solvent. The appropriate fractions were combined and the solvent removed to give 6.01 g of crude material. This was crystallized twice from $PhCH_3$/hexane (1:4) and dried to give 2.94 g of benz[*a*]anthracene-3-carbaldehyde (28.9% yield).

7C. 2-((3-Benz[*a*]anthracenylmethyl)amino)-2-methyl-1,3-propanediol methanesulfonate

Using the reductive amination procedure outlined in Example 1 benz[*a*]anthracene-3-carbaldehyde (7B) and 2-amino-2-methyl-1,3-propanediol (Aldrich) gave 2-((3-benz[*a*]anthracenylmethyl)amino)-2-methyl-1,3-propanediol methanesulfonate in 52.7% yield, mp 189—191.5°, which analyzed correctly (C, H, N, S) for the assigned structure.

Example 8

2-[(4*H*-Cyclopenta[*def*]phenanthren-1-ylmethyl)amino]-2-methyl-1,3-propanediol

8A. 4*H*-Cyclopenta[*def*]phenanthrene-1-carbaldehyde

4*H*-cyclopenta[*def*]phenanthrene (Aldrich) was formylated using the procedure of A. Rieche et al (*Chem. Ber. 93,* 88 (1960)). The crude aldehyde was passed through a plug of silica gel using methylene chloride as the eluting solvent. The appropriate fractions were combined and the solvent removed to give 48 g of product. This was recrystallized from hexane to give 28.1 g of pure 4*H*-cyclopenta[*def*]phenanthrene-1-carbaldehyde (53.6% yield) which analyzed correctly for the assigned structure.

8B. 2-[(4*H*-Cyclopenta[*def*]phenanthren-1-ylmethyl)amino]-2-methyl-1,3-propanediol methanesulfonate

Using the reductive amination procedure outlined in Example 1, 4*H*-cyclopenta[*def*]phenanthrene-1-carbaldehyde (8A) and 2-amino-2-methyl-1,3-propanediol (Aldrich) gave 2-[(4*H*-cyclopenta[*def*]phenanthren-1-ylmethyl)amino]-2-methyl-1,3-propanediolmethanesulfonate (1/20 n-PrOH) in 10% yield, mp 160—162 which analyzed correctly (C, H, N, S) for the assigned structure.

Example 9

(2-[[(4,5-Dihydro-6-acephenanthrylenyl)methyl]amino]-2-methyl-1,3-propanediol

Using the reductive amination procedure outlined in Example 1, 4,5-dihydro-6-acephenanthrylenecarbaldehyde (J. P. Hoeffinger, P. Jacquignon, and N. P. Buu-Hol, *Bull. Soc. Chim. Fr.* 974 (1970)) and 2-amino-2-methyl-1,3-propanediol (Aldrich) gave (2-[[(4,5-dihydro-6-acephenanthrylenyl)methyl]amino]-2-methyl-1,3-propanediol methanesulfonate in 74% yield, mp 240—242° (dec), which analyzed correctly (C, H, N, S) for the assigned structure.

Example 10

2-[(6-Acephenanthrylenylmethyl)amino)-2-methyl-1,3-propanediol

10A. 6-Acephenanthrylenecarbaldehyde

Using the dehydrogenation procedure outlined in Example 7B, 4,5-dihydro-6-acephenanthrylenecarbaldehyde (J. P. Hoeffinger, P. Jacquignon, and N. P. Buu-Hol, *Bull. Soc. Chim. Fr. 974* (1970)) gave 6-acephenanthrylenecarbaldehyde in 35.8% yield, mp which analyzed correctly (C, H) for the assigned structure.

10B. 2-[(6-Acephenanthrylenylmethyl)amino)-2-methyl-1,3-propanediolmethanesulfonate

Using the reductive amination procedure outlined in Example 1, 6-acephenanthrylenecarbaldehyde (10A) and 2-amino-2-methyl-1,3-propanediol (Aldrich) gave 2-[(6-acephenanthrylenylmethyl)amino)-2-methyl-1,3-propanediol methanesulfonate in 61.5% yield, mp 232—233 (dec), which analyzed correctly (C, H, N, S) for the assigned structure.

Example 11

In vitro antifungal activity

The minimum fungistatic concentrations of compounds of the formula (I) against various fungi were obtained using standard techniques. The tests were carried out in either medium A or medium B (see below) and the standard used in each instance was Miconazole nitrate.

| Organism | Minimum Fungistatic Concentration (µg/ml) | |
|---|---|---|
| | Compound of Example 5 | Miconazole Nitrate |
| Saccharomyces cerevisiae | ≤0.4 | ≤0.4 |
| Aspergillus fumigatus | " | " |
| Fusarium solani | " | " |
| Microsporum canis | " | " |
| Microsporum gypsum | " | " |
| Trichophyton equinum | " | " |
| Trichophyton mentagrophytes | " | " |
| Trichophyton rubrum | " | " |
| Epidermophyton floccosum | " | " |
| Sporothrix schenckii | " | " |
| Exophiala werneckii | " | " |

**Medium A :** Difco Yeast Nitrogen Base supplemented with L-asparagine and D-glucose

**Medium B :** Difco Yeast Nitrogen Base supplemented with L-asparagine, D-glucose, casein hydrolysate, and Difco Agar Noble.

**Claims for the Contracting States: BE CH DE FR GB IT LI LU NL SE**

1. A compound of the formula (I)

$$RCH_2NHR^1 \quad (I)$$

or a monomethyl or monoethyl ether thereof, the compound of formula (I) including the said ethers containing no more than 30 carbon atoms in total; an ester thereof; a salt thereof; wherein

R is a $C_{15-22}$ carbocyclic fused ring system containing 3, 4 or 5 aromatic rings optionally substituted by one or two subtituents, said substituents containing not more than four carbon atoms in total when taken together being the same or different and are selected from halogen; cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(O)_nR^2$ wherein n is an integer 0, 1 or 2 and $R^2$ is $C_{1-2}$ alkyl optionally substituted by hydroxy or $C_{1-2}$ alkoxy; or R is optionally substituted by a group $NR^3R^4$ containing not more than 5 carbon atoms wherein $R^3$ and $R^4$ are the same or different and each is a $C_{1-3}$ alkly group or $NR^3R^4$ forms a five- or six-membered heterocyclic ring optionally containing one or two additional heteroatoms;

$R^1$ contains not more than eight carbon atoms and is a group

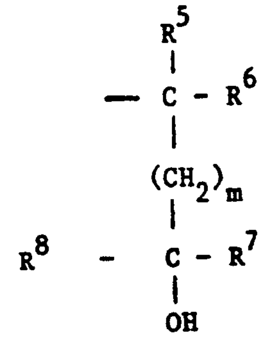

or

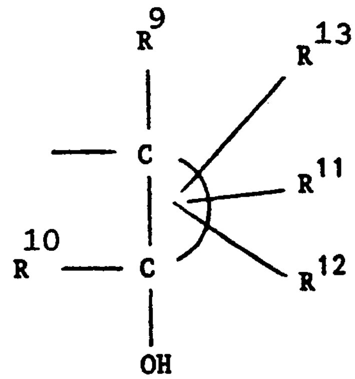

wherein

m is 0 or 1;

$R^5$ and $R^6$ are the same or different and each is hydrogen or $C_{1-5}$ alkyl optionally substituted by hydroxy;

$R^7$ and $R^8$ are the same or different and each is hydrogen or $C_{1-3}$ alkly; $\overset{\frown}{—C—C—}$ is a five- or six-membered saturated carbocyclic ring;

$R^9$ is hydrogen, methyl or hydroxymethyl;

$R^{10}$, $R^{11}$ and $R^{12}$ are the same or different and each is hydrogen or methyl;

$R^{13}$ is hydrogen, methyl, hydroxy, or hydroxymethyl;

with the proviso that R is not substituted or unsubstituted perylene, fluoranthene, chrysene, pyrene or triphenylene.

2. A compound of the formula (I) according to claim 1 in which R is selected from:

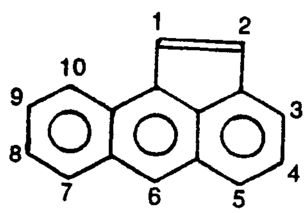

aceanthrylene

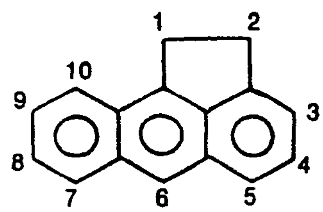

aceanthrene

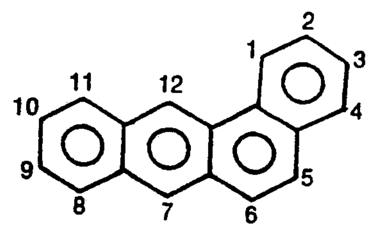

benz[*a*]anthracene

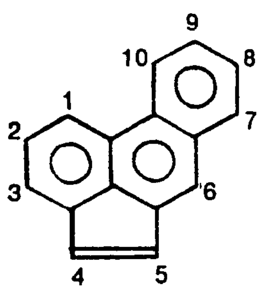

acephenanthrylene

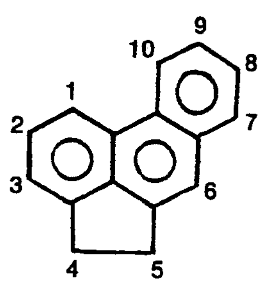

acephenanthrene

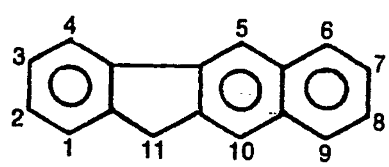

11H-benzo[*b*]fluorene

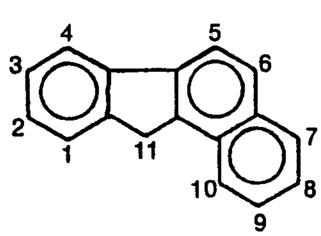

11H-benzo[*a*]fluorene

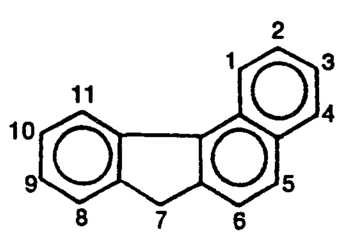

7H-benzo[*c*]fluorene

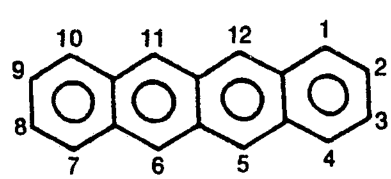

naphthacene

3. A compound of the formula (I) according to either claim 1 or 2 in which R is selected from 7-benz[*a*]anthracane, 5-naphthacene, 5-(11H-benzo[*b*]fluorene), 6-acephenanthrylene or 6-acephenanthrene.

4. A compound of the formula (I) according to any one of claims 1 to 3 in which $R^1$ is

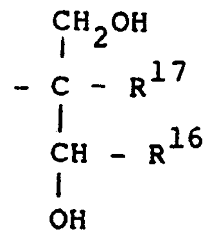

wherein $R^{16}$ is hydrogen or methyl and $R^{17}$ is hydrogen, methyl or ethyl, most preferably methyl.

5. A compound of the formula (I) according to claim 1 selected from the group comprising:
2-[(7-Benz[*a*]anthracenylmethyl)amino]-2-methyl-1,3-propanediol,
2-[(5-Naphthacenylmethyl)amino]-2-methyl-1,3-propanediol,
2-[(11*H*-Benzo[*b*]fluoren-5-ylmethyl)amino]-2-methyl-1,3-propanediol,
2-[(6-Acephenanthrylenylmethyl)amino]-2-methyl-1,3-propanediol and
2-[(6-Acephenanthrenylmethyl)amino]-2-methyl-1,3-propanediol and ethers, esters and acid addition salts thereof.

6. Pharmaceutical formulation comprising a compound of the formula (I), or an ether, ester or pharmaceutically acceptable salt thereof, together with a pharmaceutically acceptable carrier therefor.

7. A method for the preparation of a compound of the formula (I), as defined in claim 1, which method comprises:

(a) The reduction of a compound $RCH{=}NR^1$, or an appropriately protected derivative thereof, followed by deprotection where appropriate; or

(b) The reduction of a compound $R.CO.NHR^1$ wherein the hydroxy groups are optionally protected, followed by deprotection where appropriate; or

(c) The reaction of a compound $RCH_2L$, wherein L is a leaving group, with a compund $NH_2R^1$.

8. Compound of the formula I or an ether, ester or acid addition salt thereof as defined in claim 1 for use as a medicament.

9. A compound of the formula (I) or an ester, ether or acid addition salt thereof as defined in claim 1, for use as an antifungal agent.

10. A compound of the formula (I) or an ether, ester or acid addition salt thereof as defined in claim 1, for use in the preparation of a medicament for the treatment of tumors.

**Claims for the Contracting State: AT**

1. A process for the preparation of a compound of the formula (I):

$$RCH_2NHR^1 \qquad (I)$$

or a monomethyl or monoethyl ether thereof, the compound of formula (I) including the said ethers containing no more than 30 carbon atoms in total; an ester thereof; a salt thereof;
wherein

R is a $C_{15-22}$ carbocyclic fused ring system containing 3, 4 or 5 aromatic rings optionally substituted by one or two substituents, said substituents containing not more than four carbon atoms in total when taken together being the same or different and are selected from halogen; cyano $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(O)_nR^2$ wherein n is an integer 0, 1 or 2 and $R^2$ is $C_{1-2}$ alkyl optionally substituted by hydroxy or $C_{1-2}$ alkoxy; or R is optionally substituted by a group $NR^3R^4$ containing not more than 5 carbon atoms wherein $R^3$ and $R^4$ are the same or different and each is a $C_{1-3}$ alkly group or $NR^3R^4$ forms a five- or six-membered heterocyclic ring optionally containing one or two additional heteroatoms;

$R^1$ contains not more than eight carbon atoms and is a group

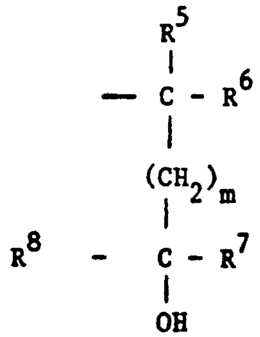

or

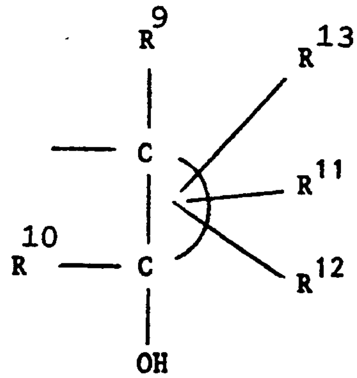

wherein

m is 0 or 1;

$R^5$ and $R^6$ are the same or different and each is hydrogen or $C_{1-5}$ alkyl optionally substituted by hydroxy;

$R^7$ and $R^8$ are the same or different and each is hydrogen or $C_{1-3}$ alkyl; $\overset{\frown}{—C—C—}$ is a five- or six-membered saturated carbocyclic ring;

$R^9$ is hydrogen, methyl or hydroxymethyl;

$R^{10}$, $R^{11}$ and $R^{12}$ are the same or different and each is hydrogen or methyl;

$R^{13}$ is hydrogen, methyl, hydroxy, or hydroxymethyl;

with the proviso that R is not substituted or unsubstituted perylene, fluoranthene, chrysene, pyrene or triphenylene.

which process comprises:

(a) The reduction of a compound $RCH{=}NR^1$, or an appropriately protected derivative thereof, followed by deprotection where appropriate: or

(b) The reduction of a compound $R.CO.NHR^1$ wherein the hydroxy groups are optionally protected, followed by deprotection where appropriate; or

(c) The reaction of a compund $RCH_2L$, wherein L is a leaving group, with a compound $NH_2R^1$.

2. A process according to claim 1 for preparing a compound of the formula (I) wherein R is selected from:

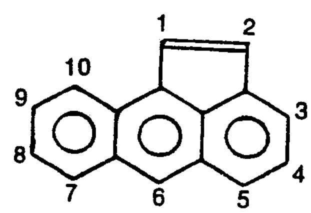

aceanthrylene

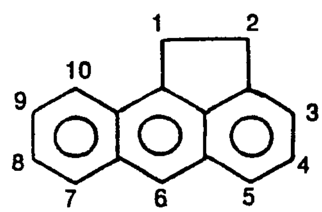

aceanthrene

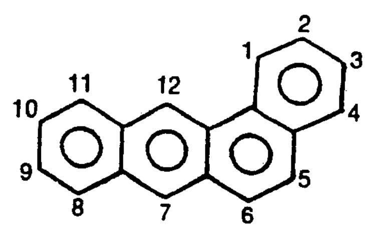

benz[*a*]anthracene

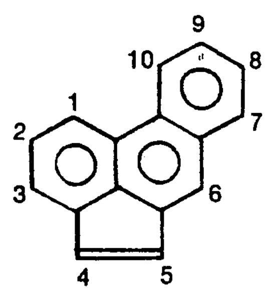

acephenanthrylene

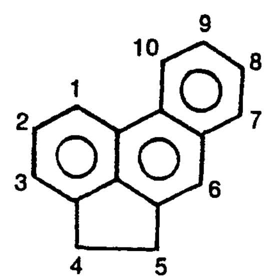

acephenanthrene

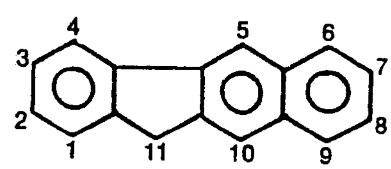

11H-benzo[*b*]fluorene

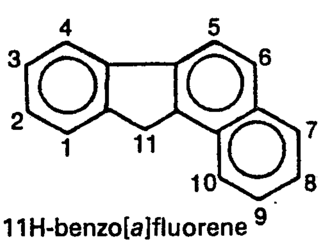

11H-benzo[*a*]fluorene

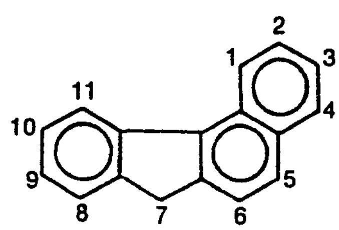

7H-benzo[*c*]fluorene

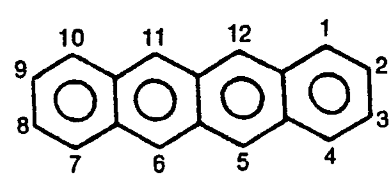

naphthacene

3. A process according to either of claims 1 or 2 for preparing a compound of the formula (I) in which R is selected from 7-benz[*a*]anthracene, 5-naphthacene, 5-[11*H*-benzo[*b*]fluorene), 6-acephenanthrylene or 6-acephenanthrene.

4. A process according to any of claims 1 to 3 for preparing a compound of the formula (1) in which $R^1$ is

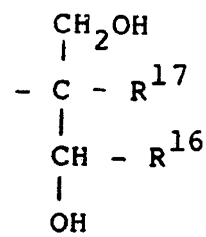

wherein $R^{16}$ is hydrogen or methyl and $R^{17}$ is hydrogen, methyl or ethyl.

5. A process according to any of claims 1 to 4 for preparing a compound selected from the group comprising:

2-[(7-Benz[*a*]anthracenylmethyl)amino]-2-methyl-1,3-propanediol,
2-[(5-Naphthacenylmethyl)amino]-2-methyl-1,3-propanediol,
2-[(11*H*-Benzo[*b*]fluoren-5-ylmethyl)amino]-2-methyl-1,3-propanediol,
2-[(6-Acephenanthrylenylmethyl)amino]-2-methyl-1,3-propanediol and
2-[(6-Acephenanthrenylmethyl)amino]-2-methyl-1,3-propanediol ethers, esters and acid addition salts thereof.

6. Pharmaceutical formulation comprising a compound of the formula (I), or an ether, ester or pharmaceutically acceptable salt thereof, together with a pharmaceutically acceptable carrier therefor.

**Patentansprüche für die Vertragsstaaten: BE CH DE FR GB IT LI LU NL SE**

1. Verbindung der Formel

$$RCH_2NHR^1 \qquad (I)$$

oder deren Monomethyl- oder Monoethylether, wobei die Verbindung der Formel (I) einschließlich der Ether, insgesamt nicht mehr als 30 Kohlenstoffatome enthält; deren Ester, deren Salz, in der

R ein $C_{15-22}$ carbocyclisch kondensiertes Ringsystem ist, das 3, 4 oder 5 aromatische Ringe enthält, die gegebenenfalls durch einen oder zwei Substituenten substituiert sind, wobei die Substituenten, wenn zusammengenommen, insgesamt nicht mehr als 4 Kohlenstoffatome enthalten und gleich oder verschieden sind und aus Halogen; Cyano; $C_{1-4}$-Alkyl oder $C_{1-4}$-Alkoxy, wobei jeder Rest gegebenenfalls durch Hydroxy oder $C_{1-2}$-Alkoxy substituiert ist; halogensubstituiertem $C_{1-2}$-Alkyl oder $C_{1-2}$-Alkoxy; einer Gruppe $S(O)_nR^2$, in der n 0, 1 oder 2 ist und $R^2$ $C_{1-2}$-Alkyl ist, das gegebenenfalls durch Hydroxy oder $C_{1-2}$-Alkoxy substituiert, ausgewählt ist, oder R ist gegebenenfalls durch eine Gruppe $NR^3R^4$, die nicht mehr als 5 Kohlenstoffatome enthält, substituiert, in der $R^3$ und $R^4$ gleich oder verschieden sind und jeder Rest eine $C_{1-3}$-Alkylgruppe ist, oder $NR^3R^4$ bildet einen 5-oder 6-gliedrigen heterocyclischen Ring, der gegebenenfalls ein oder zwei zusätzliche Heteroatome enthält;

$R^1$ nicht mehr als 8 Kohlenstoffatome enthält und eine der folgenden Gruppen ist:

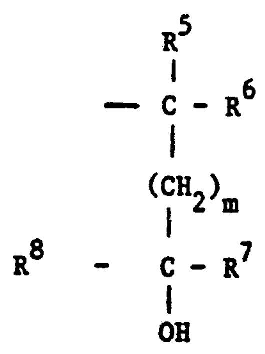

oder

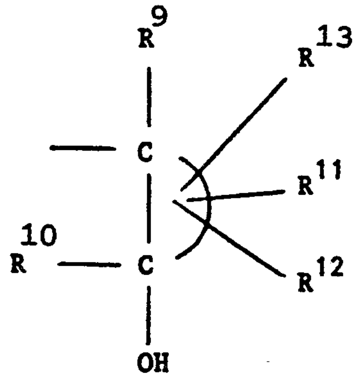

in der m 0 oder 1 ist;

$R^5$ und $R^6$ gleich oder verschieden sind und jeder Rest Wasserstoff oder $C_{1-5}$-Alkyl, gegebenenfalls durch Hydroxy substituiert, ist;

$R^7$ und $R^8$ gleich oder verschieden sind und jeder Rest Wasserstoff oder $C_{1-3}$-Alkyl ist;
—C—C— ein fünf- oder sechsgliedriger, gesättigter carbocyclischer Ring ist;
$R^9$ Wasserstoff, Methyl oder Hydroxymethyl ist;
$R^{10}$, $R^{11}$ und $R^{12}$ gleich oder verschieden sind und jeder Rest Wasserstoff oder Methyl ist;
$R^{13}$ Wasserstoff, Methyl, Hydroxy oder Hydroxymethyl ist;
mit der Maßgabe, daß R nicht substituiertes oder unsubstituiertes Perylen, Fluoranthen, Chrysen, Pyren oder Triphenylen ist.

2. Verbindung der Formel (I) nach Anspruch 1, in der R ausgewählt ist aus:

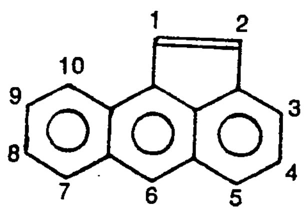
aceanthrylen

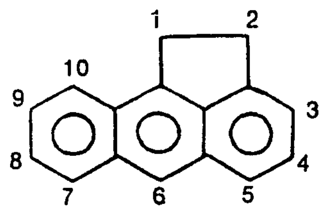
aceanthren

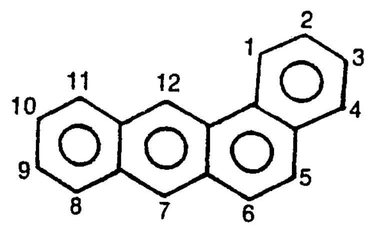
benz[*a*]anthracen

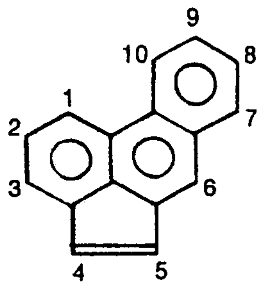
acephenanthrylen

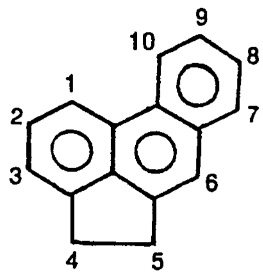
acephenanthren

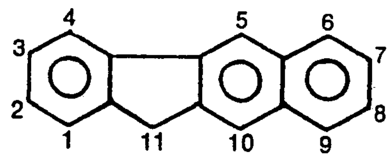
11H-benzo[*b*]fluoren

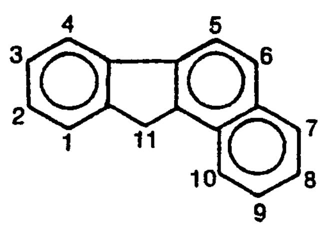
11H-benzo[*a*]fluoren

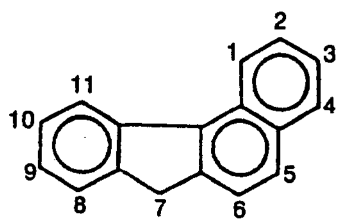
7H-benzo[*c*]fluoren

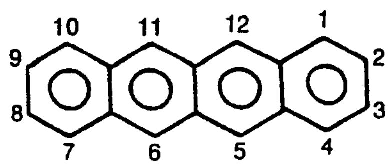
naphthacen

3. Verbindung der Formel (I) nach Anspruch 1 oder 2, in der R aus 7-Benz[a]anthracan, 5-Naphthacen, 5-(11H-Benzo[b]fluoren), 6-Acephenanthrylen oder 6-Acephenanthren ausgewählt ist.

4. Verbindung der Formel (I) nach einem der Ansprüche 1 bis 3, in der $R^1$ die folgende Gruppe ist:

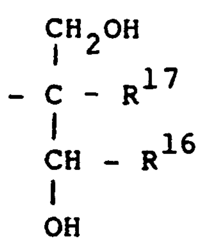

in der $R^{16}$ Wasserstoff oder Methyl ist und $R^{17}$ Wasserstoff, Methyl oder Ethyl, am bevorzugtesten Methyl, ist.

5. Verbindung der Formel (I) nach Anspruch 1, ausgewählt aus der Gruppe enthaltend:
2-[(7-Benz[*a*]anthracenylmethyl)amino]-2-methyl-1,3-propandiol,
2-[(5-Naphthacenylmethyl)amino]-2-methyl-1,3-propandiol,
2-[(11*H*-Benzo[*b*]fluoren-5-ylmethyl)amino]-2-methyl-1,3-propandiol,
2-[(6-Acephenanthrylenylmethyl)amino]-2-methyl-1,3-propandiol und

2-[(6-Acephenanthrenylmethyl)amino]-2-methyl-1,3-propandiol und deren Ether, Ester und Säureadditionssalze.

6. Pharmazeutische Formulierung, enthaltend eine Verbindung der Formel (I) oder deren Ether, Ester oder pharmazeutisch annehmbares Salz, zusammen mit einem dafür pharmazeutisch annehmbaren Trägermaterial.

7. Verfahren zur Herstellung einer Verbindung der Formel (I), wie in Anspruch 1 definiert, bei dem man:

(a) eine Verbindung $RCH{=}NR^1$ oder ein entsprechend geschütztes Derivat davon reduziert, anschließend, wo erforderlich, den Schutz entfernt, oder

(b) eine Verbindung $R.CO.NHR^1$, in der die Hydroxygruppen gegebenenfalls geschützt sind, reduziert, anschließend, wo erforderlich, den Schutz entfernt, oder

(c) eine Verbindung $RCH_2L$, in der L eine austretende Gruppe ist, mit einer Verbindung $NH_2R^1$ umsetzt.

8. Verbindung der Formel (I) oder deren Ether, Ester oder Säureadditionssalz, wie in Anspruch 1 definiert, zur Verwendung als Medikament.

9. Verbindung der Formel (I) oder deren Ester, Ether oder Säureadditionssalz, wie in Anspruch 1 definiert, zur Verwendung als Antipilzmittel.

10. Verbindung der Formel (I) oder deren Ether, Ester oder Säureadditionssalz, wie in Anspruch 1 definiert, zur Verwendung in der Herstellung eines Medikaments zur Behandlung von Tumoren.

**Patentansprüche für den Vertragsstaat: AT**

1. Verfahren zur Herstellung einer Verbindung der Formel (I):

$$RCH_2NHR^1 \qquad (I)$$

oder deren Monomethyl- oder Monoethylether, wobei die Verbindung der Formel (I) einschließlich der Ether, nicht mehr als 30 Kohlenstoffatome insgesamt enthält; deren Ester, deren Salz, in der

R ein $C_{15-22}$ carbocyclisch kondensiertes Ringsystem ist, das 3, 4 oder 5 aromatische Ringe enthält, die gegebenenfalls durch einen oder zwei Substituenten substituiert sind, wobei die Substituenten, wenn zusammengenommen, insgesamt nicht mehr als 4 Kohlenstoffatome enthalten und gleich oder verschieden sind und aus Halogen; Cyano; $C_{1-4}$-Alkyl oder $C_{1-4}$-Alkoxy, wobei jeder Rest gegebenenfalls durch Hydroxy oder $C_{1-2}$-Alkoxy substituiert ist; halogensubstituiertem $C_{1-2}$-Alkyl oder $C_{1-2}$-Alkoxy; einer Gruppe $S(O)_nR^2$, in der n 0, 1 oder 2 ist und $R^2$ $C_{1-2}$-Alkykl ist, das gegebenenfalls durch Hydroxy oder $C_{1-2}$-Alkoxy substituiert, ausgewählt ist, oder R ist gegebenenfalls durch eine Gruppe $NR^3R^4$, die nicht mehr als 5 Kohlenstoffatome enthält, substituiert, in der $R^3$ und $R^4$ gleich oder verschieden sind und jeder Rest eine $C_{1-3}$-Alkylgruppe ist, oder $NR^3R^4$ bildet einen 5-oder 6-gliedrigen heterocyclischen Ring, der gegebenenfalls ein oder zwei zusätzliche Heteroatome enthält;

$R^1$ nicht mehr als 8 Kohlenstoffatome enthält und eine der folgenden Gruppen ist:

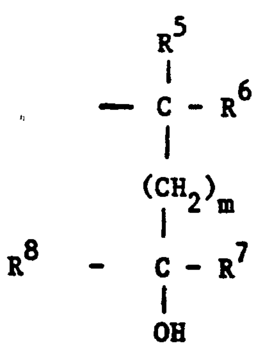

oder

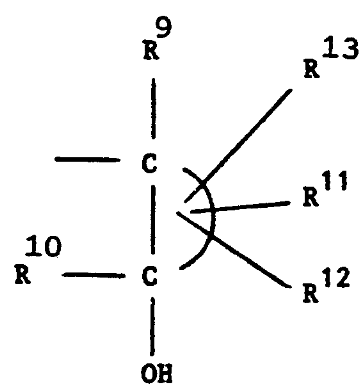

in der m 0 oder 1 ist;

$R^5$ und $R^6$ gleich oder verschieden sind und jeder Rest Wasserstoff oder $C_{1-5}$-Alkyl, gegebenenfalls durch Hydroxy substituiert, ist;

$R^7$ und $R^8$ gleich oder verschieden sind und jeder Rest Wasserstoff oder $C_{1-3}$-Alkyl ist;

—C—C— ein fünf- oder sechsgliedriger, gesättigter carbocyclischer Ring ist;

$R^9$ Wasserstoff, Methyl oder Hydroxymethyl ist;

$R^{10}$, $R^{11}$ und $R^{12}$ gleich oder verschieden sind und jeder Rest Wasserstoff oder Methyl ist;

$R^{13}$ Wasserstoff, Methyl, Hydroxy oder Hydroxymethyl ist;

mit der Maßgabe, daß R nicht substituiertes oder unsubstituiertes Perylen, Fluoranthen, Chrysen, Pyren oder Triphenylen ist, bei dem man:

(a) eine Verbindung $RCH{=}NR^1$ oder ein entsprechend geschütztes Derivat davon reduziert, anschließend, wo erforderlich, den Schutz entfernt, oder

(b) eine Verbindung R.CO.NHR$^1$, in der die Hydroxygruppen gegebenenfalls geschützt sind, reduziert, anschließend, wo erforderlich, den Schutz entfernt, oder

(c) eine Verbindung $RCH_2L$, in der L eine austretende Gruppe ist, mit einer Verbindung $NH_2R^1$ umsetzt.

2. Verfahren nach Anspruch 1 zur Herstellung einer Verbindung der Formel (I), bei dem R ausgewählt wird aus:

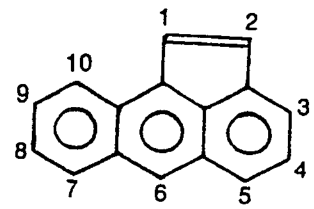

aceanthrylen

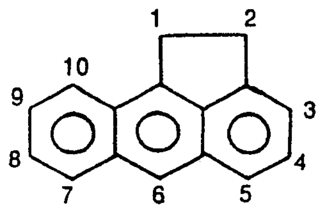

aceanthren

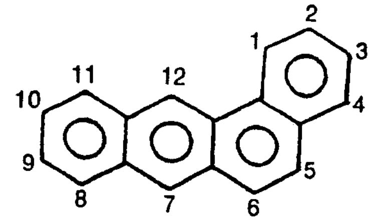

benz[*a*]anthracen

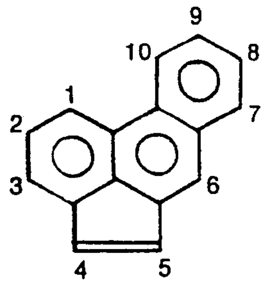

acephenanthrylen

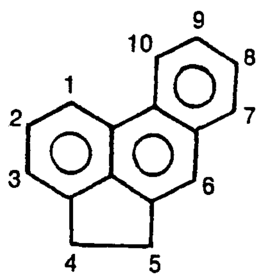

acephenanthren

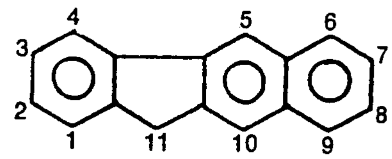

11H-benzo[*b*]fluoren

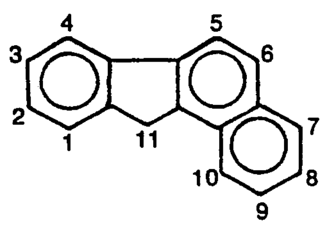

11H-benzo[*a*]fluoren

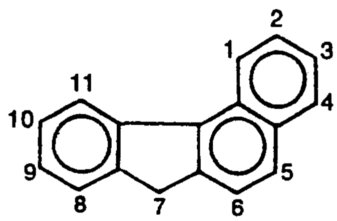

7H-benzo[*c*]fluoren

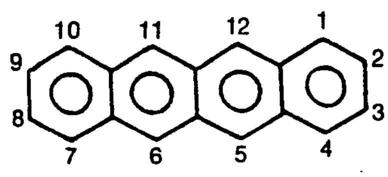

naphthacen

3. Verfahren nach einem der Ansprüche 1 oder 2 zur Herstellung einer Verbindung der Formel (I), in der R aus 7-Benz[*a*]anthracen, 5-Naphthacen, 5-(11*H*-benzo[b]fluoren), 6-Acephenanthrylen oder 6-Acephenanthren, ausgewählt wird.

4. Verfahren nach einem der Ansprüche 1 bis 3 zur Herstellung einer Verbindung der Formel (I), in der R$^1$

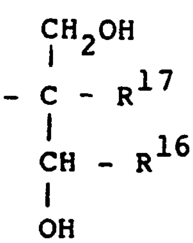

ist, worin R$^{16}$ Wasserstoff oder Methyl ist und R$^{17}$ Wasserstoff, Methyl oder Ethyl, ist.

5. Verfahren nach einem der Ansprüche 1 bis 4 zur Herstellung einer Verbindung, ausgewählt aus der Gruppe enthaltend:

2-[(7-Benz[*a*]anthracenylmethyl)amino]-2-methyl-1,3-propandiol,

2-[(5-Naphthacenylmethyl)amino]-2-methyl-1,3-propandiol,

2-[(11*H*-Benzo[*b*]fluoren-5-ylmethyl)amino]-2-methyl-1,3-propandiol,

2-[(6-Acephenanthrylenylmethyl)amino]-2-methyl-1,3-propandiol und

2-[(6-Acephenanthrenylmethyl)amino]-2-methyl-1,3-propandiol und deren Ether, Ester und Säureadditionssalze.

6. Pharmazeutische Formulierung, enthaltend eine Verbindung der Formel (I) oder einen Ether, Ester

oder pharmazeutisch annehmbares Salz davon, zusammen mit einem pharmazeutisch annehmbaren Trägermaterial dafür.

**Revendications pour les Etats contractants: BE CH DE FR GB IT LI LU NL SE**

1. Composé de formule (I)

$$RCH_2NHR^1 \qquad (I)$$

ou un éther monométhylique ou monoéthylique de celui-ci, le composé de formule (I) incluant ces éthers qui ne contiennent pas plus de 30 atomes de carbone au total; un ester de celui-ci ou un sel de celui-ci;

où R est un système carbocyclique condensé en $C_{15-22}$ contenant 3, 4 ou 5 cycles aromatiques éventuellement substitués par un ou deux substituants, ces substituants ne comptant pas plus de quatre atomes de carbone au total lorsqu'ils sont pris ensemble et étant identiques ou différents et choisis parmi halogéno; cyano; $C_{1-4}$-alcoyle ou $C_{1-4}$-alcoxy, chacun éventuellement substitué par hydroxyle ou $C_{1-2}$-alcoxy; $C_{1-2}$-alcoyle ou $C_{1-2}$-alcoxy halogéno-substitué; un radical $S(O)_nR^2$ où n est un entier valant 0, 1 ou 2 et $R^2$ est $C_{1-2}$-alcoyle éventuellement substitué par hydroxyle ou $C_{1-2}$-alcoxy; ou bien R est éventuellement substitué par un radical $NR^3R^4$ ne comptant pas plus de 5 atomes de carbone, où $R^3$ et $R^4$ sont identiques ou différents et sont chacun un radical $C_{1-3}$-alcoyle ou bien $NR^3R^4$ forme un hétérocycle de cinq ou six chaînons contenant éventuellement un ou deux hétéroatomes supplémentaires;

$R^1$ ne compte pas plus de huit atomes de carbone et est un radical

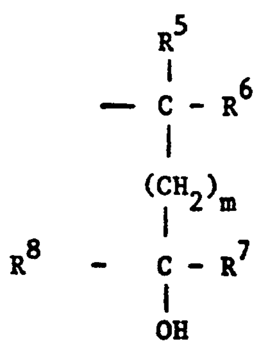

ou

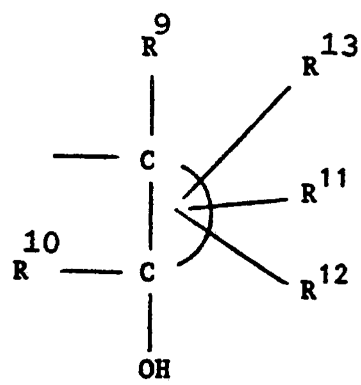

où m est 0 ou 1; $R^5$ et $R^6$ sont identiques ou différents et sont chacun hydrogène ou $C_{1-5}$-alcoyle éventuellement substitué par hydroxyle; $R^7$ et $R^8$ sont identiques ou différents et sont chacun hydrogène ou $C_{1-3}$-alcoyle;

$-\overset{\frown}{C-C}-$ est un carbocycle saturé de cinq ou six chaînons; $R^9$ est hydrogène, méthyle ou hydroxyméthyle; $R^{10}$, $R^{11}$ et $R^{12}$ sont identiques ou différents et sont chacun hydrogène ou méthyle; $R^{13}$ est hydrogène, méthyle, hydroxyle ou hydroxyméthyle;
avec la restriction que R n'est pas pérylène, fluoranthène, chrysène, pyrène ou triphénylène substitué ou non substitué.

2. Composé de formule (I) suivant la revendication 1, où R est choisi parmi:

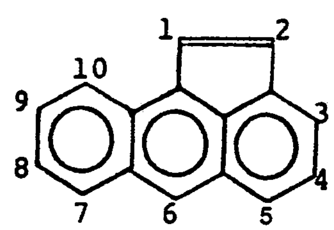

acéanthrylène

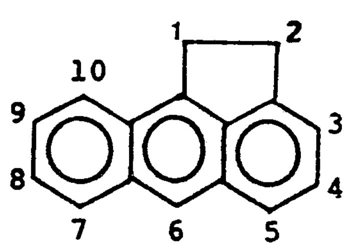

acéanthrène

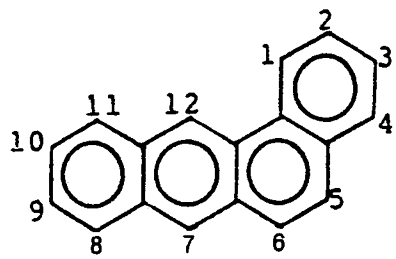

benz[a]anthacène

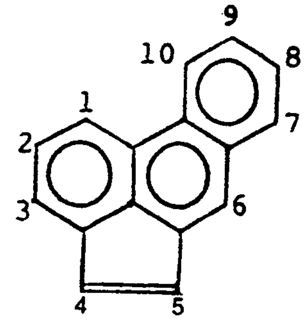

acéphénanthrylène

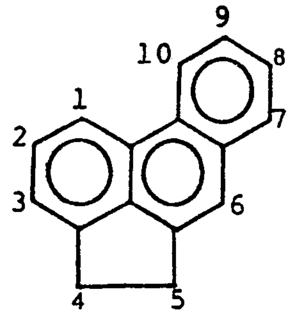

acéphénanthrène

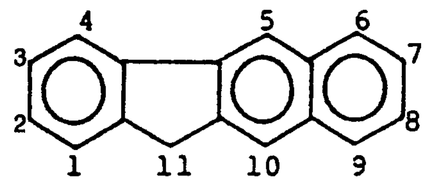

11H-benzo[b]fluorène

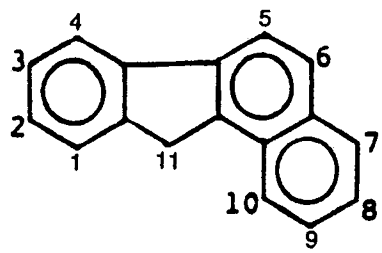

11H-benzo[*a*]fluorène

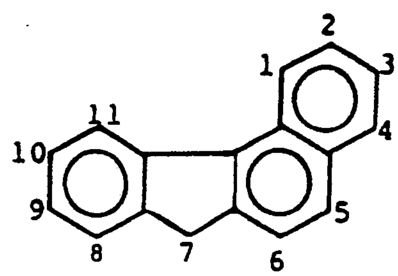

7H-benzo[c]fluorène

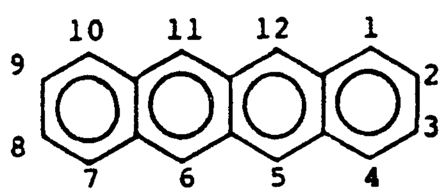

naphtacène

3. Composé de formule (I) suivant la revendication 1 ou 2, dans lequel R est choisi parmi 7-benz[a]anthracène, 5-naphtacène, 5-(11H-benzo[b]fluorène), 6-acéphénanthrylène et 6-acéphénanthrène.

4. Composé de formule (I) suivant l'une quelconque des revendications 1 à 3, dans lequel $R^1$ est

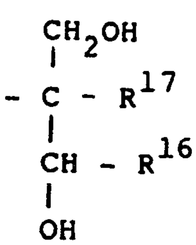

où $R^{16}$ est hydrogène ou méthyle et $R^{17}$ est hydrogène, méthyle ou éthyle, mais de préférence méthyle.

5. Composé de formule (I) suivant la revendication 1, choisi dans la classe formée par:

le 2-[(7-benz[*a*]anthracénylméthyl)amino]-2-méthyl-1,3-propanediol,

le 2-[(5-naphtacénylméthyl)amino]-2-méthyl-1,3-propanediol,
le 2-[(11*H*-benzo[*b*]fluorén-5-ylméthyl)amino]-2-méthyl-1,3-propanediol,
le 2-[(6-acéphénanthrylénylméthyl)amino]-2-méthyl-1,3-propanediol,
le 2-[(6-acéphénanthrénylméthyl)amino]-2-méthyl-1,3-propanediol, et
les éthers, esters et sels d'addition d'acides de ceux-ci.

6. Composition pharmaceutique comprenant un composé de formule (I) ou un éther, ester ou sel pharmaceutiquement acceptable de celui-ci, conjointement avec un excipient pharmaceutiquement acceptable.

7. Procédé de préparation d'un composé de formule (I) tel que défini dans la revendication 1, lequel procédé comprend:

(a) La réduction d'un composé $RCH{=}NR^1$ ou d'un dérivé convenablement protégé de celui-ci, suivie de la déprotection s'il y a lieu; ou

(b) La réduction d'un composé $R.CO.NHR^1$ où les radicaux hydroxyle sont éventuellement protégés, suivie de la déprotection s'il y a lieu, ou

(c) La réaction d'un composé $RCH_2L$, où L est un radical partant, avec un composé $NH_2R^1$.

8. Composé de formule I ou un éther, ester ou sel d'addition d'acide de celui-ci tel que défini dans la revendication 1, à utiliser comme médicament.

9. Composé de formule (I) ou un ester, éther ou sel d'addition d'acide de celui-ci tel que défini dans la revendication 1, à utiliser comme agent antifongique.

10. Composé de formule (I) ou un éther, ester ou sel d'addition d'acide de celui-ci tel que défini dans la revendication 1, à utiliser dans la préparation d'un médicament pour le traitement de tumeurs.

**Revendications pour l'Etat contractant: AT**

1. Procédé préparation d'un composé de formule (I)

$$RCH_2NHR^1 \qquad (I)$$

ou d'un éther monométhylique ou monoéthylique de celui-ci, le composé de formule (I) incluant ces éthers ne comptant pas plus de 30 atomes de carbone au total, d'un ester de celui-ci ou d'un sel de celui-ci;

où R est un système carbocyclique condensé en $C_{15-22}$ contenant 3, 4 ou 5 cycles aromatiques éventuellement substitués par un ou deux substituants, ces substituants ne comptant pas plus de quatre atomes de carbone au total lorsqu'ils sont pris ensemble et étant identiques ou différents et choisis parmi halogéno; cyano; $C_{1-4}$-alcoyle ou $C_{1-4}$-alcoxy, chacun éventuellement substitué par hydroxyle ou $C_{1-2}$-alcoxy; $C_{1-2}$-alcoyle ou $C_{1-2}$-alcoxy halogéno-substitué; un radical $S(O)_nR^2$ où n est un entier valant 0, 1 ou 2 et $R^2$ est $C_{1-2}$-alcoyle éventuellement substitué par hydroxyle ou $C_{1-2}$-alcoxy; ou bien R est éventuellement substitué par un radical $NR^3R^4$ ne comptant pas plus de 5 atomes de carbone, où $R^3$ et $R^4$ sont identiques ou différents et sont chacun un radical $C_{1-3}$-alcoyle ou bien $NR^3R^4$ forme un hétérocycle de cinq ou six chaînons contenant éventuellement un ou deux hétéroatomes supplémentaires;

$R^1$ ne compte pas plus de huit atomes de carbone et est un radical

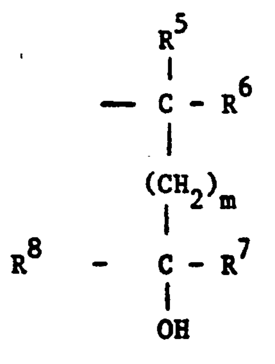

ou

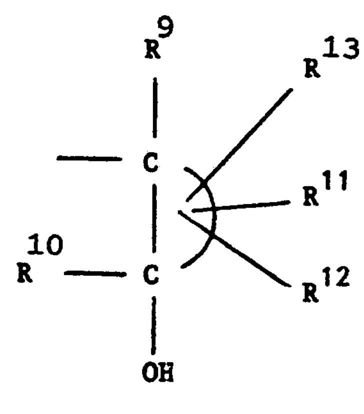

où m est 0 ou 1; $R^5$ et $R^6$ sont identiques ou différents et sont chacun hydrogène ou $C_{1-5}$-alcoyle éventuellement substitué par hydroxyle; $R^7$ et $R^8$ sont identiques ou différents et sont chacun hydrogène ou $C_{1-3}$-alcoyle; —C—C— est un carbocycle saturé de cinq ou six chaînons; $R^9$ est hydrogène, méthyle ou hydroxyméthyle; $R^{10}$, $R^{11}$ et $R^{12}$ sont identiques ou différents et sont chacun hydrogène ou méthyle; $R^{13}$ est hydrogène, méthyle, hydroxyle ou hydroxyméthyle;
avec la restriction que R n'est pas pérylène, fluoranthène, chrysène, pyrène ou triphénylène substitué ou non substitué, lequel procédé comprend:

(a) La réduction d'un composé $RCH{=}NR^1$ ou d'un dérivé convenablement protégé de celui-ci, suivie de la déprotection s'il y a lieu; ou

(b) La réduction d'un composé $R.CO.NHR^1$ où les radicaux hydroxyle sont éventuellement protégés,

suivie de la déprotection s'il y a lieu, ou

(c) La réaction d'un composé $RCH_2L$, où L est un radical partant, avec un composé $NH_2R^1$.

2. Procédé suivant la revendication 1 de préparation d'un composé de formule (I), dans lequel R est choisi parmi:

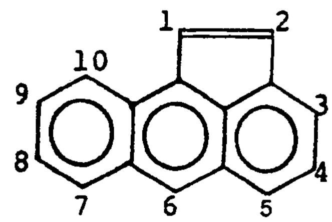

acéanthrylène

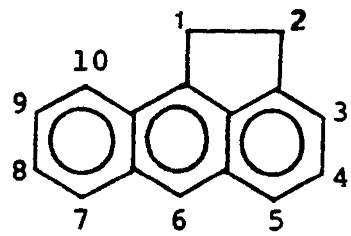

acéanthrène

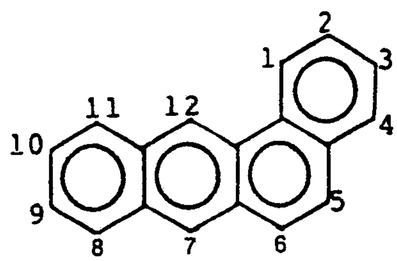

benz[a]anthacène

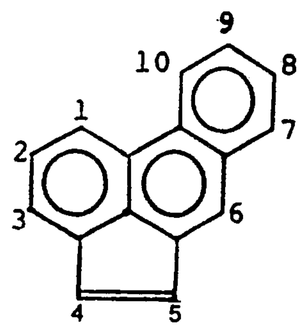

acéphénanthrylène

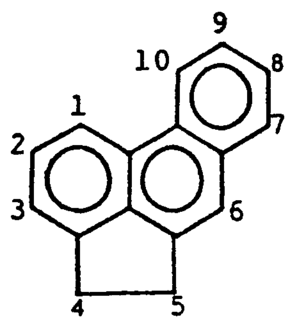

acéphénanthrène

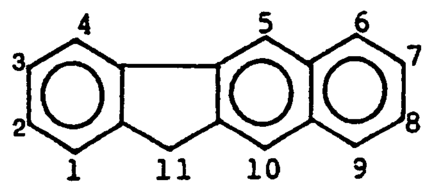

11H-benzo[b]fluorène

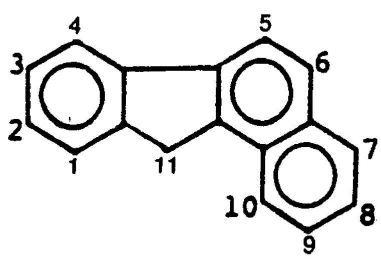

11H-benzo[*a*]fluorène

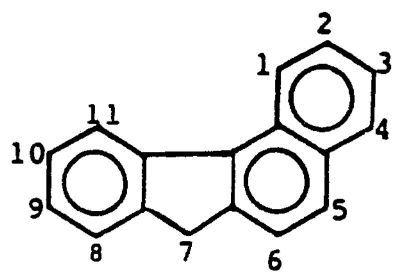

7H-benzo[c]fluorène

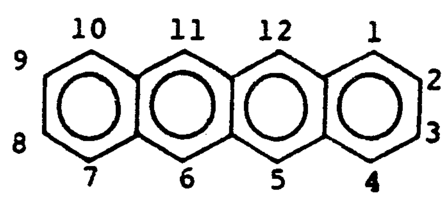

naphtacène

3. Procédé suivant l'une quelconque des revendications 1 et 2 de préparation d'un composé de formule (I), dans lequel R est choisi parmi 7-benz[a]anthracène, 5-naphtacène, 5-(11H-benzo[b]fluorène), 6-acéphénanthrylène et 6-acéphénanthrène.

4. Procédé suivant l'une quelconque des revendications 1 à 3 de préparation d'un composé de formule (I), dans lequel $R^1$ est

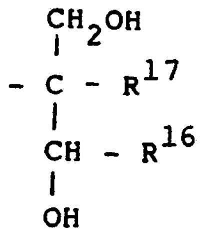

où $R^{16}$ est hydrogène ou méthyle et $R^{17}$ est hydrogène, méthyle ou éthyle.

5. Procédé suivant l'une quelconque des revendications 1 à 4 de préparation d'un composé choisi dans la classe formée par:

le 2-[(7-benz[*a*]anthracénylméthyl)amino]-2-méthyl-1,3-propanediol,
le 2-[(5-naphtacénylméthyl)amino]-2-méthyl-1,3-propanediol,
le 2-[(11*H*-benzo[*b*]fluorén-5-ylméthyl)amino]-2-méthyl-1,3-propanediol,
le 2-[(6-acéphénanthrylénylméthyl)amino]-2-méthyl-1,3-propanediol,
le 2-[(6-acéphénanthrénylméthyl)amino]-2-méthyl-1,3-propanediol, et

les éthers, esters et sels d'addition d'acides de ceux-ci.

6. Composition pharmaceutique comprenant un composé de formule (I) ou un éther, ester ou sel pharmaceutiquement acceptable de celui-ci, conjointement avec un excipient pharmaceutiquement acceptable.